# EUROPEAN PATENT SPECIFICATION

(11) **EP 0 755 468 B1**
(45) Date of publication and mention of the grant of the patent: **03.05.2000**
(21) Application number: 95902316.9
(22) Date of filing: 25.11.1994
(51) Int. Cl.: D21H 21/14

(54) **PREPARATION PROCESS OF PAPER FOR INCREASING FILLER CONTENTS AND ENHANCING SCOTT INTERNAL BOND STRENGTH**
VERFAHREN ZUR HERSTELLUNG VON PAPIER, ZUR ERHÖHUNG DES FÜLLSTOFFANTEILS UND ZUR VERBESSERUNG DER SCOTT INNENBINDUNGSFESTIGKEIT
PROCEDE DE PREPARATION DE PAPIER SERVANT A AUGMENTER LA TENEUR EN CHARGES ET A AMELIORER LA COHESION INTERNE SCOTT

(30) Priority: 12.04.1994 KR 9407591
(43) Date of publication of application: 29.01.1997
(73) Proprietor: KOREA RESEARCH INSTITUTE OF CHEMICAL TECHNOLOGY, Daejeon, 305-343 (KR)
(72) Inventor: OW, Steven, Say-Kyoun, Daejeon 302-173 (KR); SOHN, Chang, Man #301-1309 Hanshin Samcha Apt., Namyangju-Shi Kyunggi-Do (KR); HAN, Sin, Ho, Daejeon 305-328 (KR); SHIN, Jong, Ho 110-1502, Hanwool Apartment, Daejeon 305-345 (KR)
(74) Representative: Enskat, Michael Antony Frank
(86) International application number: KR9400171
(87) International publication number: WO9527825

(56) References cited:
- EP-A- 0 285 487
- EP-A- 0 326 410
- EP-A- 0 330 635

## Description

### Technical Field

The present invention relates to a preparation process of a paper for increasing the contents of inorganic fillers in the paper while maintaining the internal bond strength of the paper. The present invention further relates to a preparation process of paper wherein a wood pulp is treated with a cellulase-type enzyme for beating to thereby improve the paper strength, particularily the internal bond strength so that the filler contents of paper can be increased as much as the increased amount of the internal bond strength. As a result a paper having a large amount of fillers while maintaining an internal bond strength thereof can be prepared.

### Background Art

Generally, a printing paper is prepared by mixing a beated wood pulp with inorganic fillers and then adding at least one cohesive agent. As a fillers for paper production, talc, calcium carbonate, titanium dioxide etc. can be used.

A portion of pulp can be replaced with a filler to save the production cost of the paper and the brightness, the opacity, the smoothness and printability of the paper can be improved. Therefore, addition of a filler into paper is preferable.

However, when a large amount of a filler is added into the pulp, since the fillers weaken the intermolecular binding force of the pulp, the physical and mechanical properties of the pulp deteriorate. Further, during the drainage process of the pulp fibers on the wires of a paper machine, the fillers pass through the wires and the fillers retention in paper decreases. The wires are severely abraded and therefore the load for circulation of white water and water drain is increased, which is unpreferable. US-A-5 423 946 discloses a process wherein the drainage of the pulp is improved by treating said pulp with an cellulase type enzyme.

### Disclosure of the Invention

Therefore, it is an objective of the present invention to provide a prepartion process of paper wherein the contents of the inorganic fillers included in the paper is increased while maintaining the internal bond strength thereof.

In accordance with the present invention, a paper stock is prepared by mixing a pulp, a retention enhancing agent, an inorganic filler and a paper strength enhancing agent and treating the mixture at around 40°C at a pH of 4,0 to 6,8 with a cellulase-type enzyme, whereby the internal bond strength of the paper is enhanced which enables the content of filler to be increased. Then, this pulp is disintegrated, treated for beating and processed for paper making. At this time, due to an activity of the cellulase type enzyme on the pulp fiber, the surface area of the fiber is increased. As a result, the bond force between the fibers is increased to thereby enhance the internal bond force of the paper.

### Best Modes for carrying out the invention

In accordance with a preparation process of paper, a cellulase-type enzyme is added to a pulper to dissolve the pulp and then the pulp is beated for a preparation of paper.

The cellulase type enzyme is added to the pulper in amount of 0.01 ∼ 0.2% by weight based on the weight of the wood pulp, thereby disintegrating the wood pulp and then beating the wood pulp. At this time, as a cellulase type enzyme, an enzyme produced by *Trichoderma longibrachiatum* which has a carboxymethycellulose activity of 3,600U/mℓ or more, is preferably used.

When the beated wood pulp is processed for the preparation of paper in the same manner as a conventional process, the cellulase-type enzyme activates the surface of the pulp fiber of thereby increase the surface area of the fiber. Therefore, the binding between the fibers is improved to increase the internal bond strength of paper. Therefore, the contents of the filler can be increased as much as the increased amount of the internal binding force.

The preparation process of paper according to the present invention will be explained in detail hereinafter.

Bleached chemical wood pulp and 0.01 ∼ 0.2% by weight(based on the weight of the wood pulp)of a cellulase type enzyme having a carboxymethylcellulase activity of 3,600U/mℓ or more are simultaneously introduced into a pulper. At this time, the dissolving temperature is maintained between 35 ∼ 45°C and pH of 4.0 ∼ 6.8.

Under these conditions, the pulp is disintegrated for 15 ∼ 20 minutes and then beated using a refiner so as to have a suitable beating degree. The pulp stock thus prepared in this manner is transferred to the paper machine for paper making. The cellulase type enzyme activates the surface of the pulp fiber to increase the surface area of the fiber. As a result, the strength of paper, especially the internal bond strength is remarkably increased.

In a conventional process, a large amount of fillers can not be included in the paper since the fillers deteriorate the physical properties of the paper. However, in the present invention, due to the enzyme treatment, the internal bond strength is increased. Therefore, the percentage of filler materials may be increased as much as the increased amount of the internal bond strength without negatively affecting the final bond strength of the paper.

The paper prepared in accordance with the present invention as explained above has the following properties when compared with that prepared in accordance with a conventional process.
1. The beating power consumption can be reduced.
2. When having the same amount of fillers, the internal bond strength is improved.
3. When having the same internal bond strength, the absolute contents of fillers can be increased by up to 5% by weight.
4. As a large amount of fillers is included in the paper, the drying power consumption of the paper can be reduced.

Hereinafter, the present invention will be explained in detail with reference to the following example.

### Example 1

A pulp stock was prepared to have the composition as shown in table 1 as below.

**Table 1**

| | Components | Composition rate (% by weight) |
|---|---|---|
| Pulp | Bleached chemical pulp of needle-leaf trees | 85 |
| | Bleached chemical pulp of broad-leaf trees | 15 |
| Retention enhancing agent | Cationic polyacrylamide | 0.05 * |
| Fillers | Calcium carbonate | 15 ** |
| | Talc | 15 ** |
| Paper stength enhancing agent | Positive starch | 0.5 * |

| | | |
|---|---|---|
| * Composition ratio based on the weight of the produced paper. | | |
| ** Composition ratio based on the weight of the pulp. | | |

Using the composition ratio as shown in table 1, as a cellulase-type enzyme an enzyme produced by *Trichoderma longibrachiatum* having a carboxymethyl-cellulase activity of 3,600U/mℓ or more was introduced into a pulper in an amount of 0.02% by weight based on the pulp while maintaining a temperature of about 40°C and pH of 4.0 ∼ 6.8.

Under the above conditions, after dissolving the pulp stock for 15 minutes, the pulp stock was beated to a predetermined beating degree, to prepare a hand sheet having 80g/m² basis weight, which was conditioned at a constant humidity of 49 ∼ 51% and at a temperature of 22 ∼ 24°C. Thereafter, ash contents and Scott internal bond strength of the paper were measured. The results are shown in Table 2.

### Comparative Example 1

In order to compare the preparation process of paper according to the present invention and a conventional preparation process of paper, a paper was prepared in the same manner as in Example 1 except that an enzyme was not introduced into the pulper. Ash contents and Scott internal bond strength of the paper were measured. The results are shown in Table 2.

### Example 2

A paper was prepared in the same manner as in Example 1 except that the enzyme was introduced in the pulper in an amount of 0.1% by weight. Ash contents and Scott internal bond strength were measured and the results are shown in Table 2.

### Example 3

A paper was prepared in the same manner as in Example 1 except that the enzyme was introduced in the pulper in an amount of 0.2% by weight. Ash contents and Scott internal bond strength were measured and the results are shown in Table 2.

### Example 4

A paper was prepared in the same manner as in Example 1 except that the enzyme was introduced into the pulper in an amount of 0.1% by weight and that calcium carbonate and talc were added in an amount of 20% each by weight respectively. Ash contents and Scott internal bond strength were measured. The results are shown in Table 2.

**Table 2**

| | Ex. 1 | Ex. 2 | Ex. 3 | Ex. 4 | Comparative Example 1 |
|---|---|---|---|---|---|
| Ash contents (% by weight) | 20.2 | 20.3 | 20.3 | 24.9 | 20.1 |
| Scott internal bond strength | 149 | 167 | 173 | 135 | 134 |

As can be seen from the above, the paper prepared according to the present invention has an increased internal bond strength at the same amount of ash contents when compared to a paper prepared according to a conventional process. Further, Scott internal bond strength is remained at the same level as the conventional process in spite of an increased amount of inorganic fillers.

Therefore, the paper prepared according to the present invention maintains an internal binding strength which is one of most important characteristics for a coating base paper, a printing paper and a photocopy paper in spite of an increased amount of an inorganic filler contained in the paper. Further, as the amount of the enzyme is increased, the internal bond strength is enhanced. The amount of a pulp material can be saved as much as the increased amount of the fillers.

## Claims

1. A process for preparing a base paper of a coating paper, a printing paper or a copying paper characterized in that a paper stock is prepared by mixing a pulp, a retention enhancing agent, an inorganic filler and a paper strength enhancing agent and treating the mixture at around 40°C and at a pH of 4.0 to 6.8 with a cellulase-type enzyme, thereby enhancing the internal bond strength of the paper and enabling the content of filler to be increased.

2. The process as claimed in Claim 1, wherein the cellulase type enzyme is a Trichoderma species.

3. The process as claimed in Claim 2, wherein the cellulase-type enzyme has a carboxymethyl-cellulose activity of at least 3,600 U/ml or more, which enzyme is obtained from Trichoderma Longibrachiatum.

4. The preparation as claimed in Claim 1, wherein the cellulase-type enzyme is added to the pulp in an amount of 0.01-0.2% by weight of the pulp.

5. The process as claimed in Claim 1, wherein the inorganic filler is at least one selected from the group consisting of talc, calcium carbonate and titanium dioxide.

6. The process as claimed in Claim 1, wherein the inorganic filler is present in an amount of 30-40% by weight.

## Patentansprüche

1. Verfahren zur Herstellung des Rohpapiers eines Streichpapiers, eines Druckpapiers oder eines Kopierpapiers, dadurch gekennzeichnet, dass ein Ganz-Papierzeug durch Mischen einer Papiermasse, eines Retentionsverbesserungsmittels, eines anorganischen Füllstoffs und eines die Papierfestigkeit verbessernden Mittels hergestellt wird und Behandeln der Mischung bei ca. 40 °C und einem PH-Wert von 4,0 bis 6,8 mit einem cellulaseartigen Enzym, wodurch die Innenbindungsfestigkeit des Papiers verbessert und die Erhöhung des Füllstoffanteils ermöglicht wird.

2. Verfahren nach Anspruch 2, worin das cellulaseartige Enzym eine Trichodermaspezies ist.

3. Verfahren nach Anspruch 2, worin das cellulaseartige Enzym eine Carboxymethyl-Celluloseaktivität von mindestens 3.600 U/ml oder mehr aufweist, wobei man das Enzym aus Trichoderma-Longibrachiatum erhält.

4. Herstellung nach Anspruch 1, worin der Papiermasse ein cellulaseartiges Enzym, in einer Menge von 0,01-0,2 Gew.-% der Papiermasse, zugefügt wird.

5. Verfahren nach Anspruch 1, worin der anorganische Füllstoff mindestens einer ist, der aus der Gruppe ausgewählt wird, die aus Talk, Calciumcarbonat und Titaniumdioxid besteht.

6. Verfahren nach Anspruch 1, worin der anorganische Füllstoff in einer Menge von 30-40 Gew.-% zugegen ist.

## Revendications

1. Procédé pour d'une part préparer un papier formant la base pour un papier de revêtement, un papier d'imprimerie ou un papier à copier, **caractérisé en ce qu'**un stock de papier est préparé en mélangeant ensemble une pâte, un agent augmentant la retention, une charge inorganique et un agent augmentant la résistance du papier, et d'autre part pour traiter ce mélange à environ 40°C et à un pH de 4,0 à 6,8 avec un enzyme de type cellulasique, renforçant ainsi la résistance interne agglomérante du papier et permettant d'augmenter le contenu de charge.

2. Procédé selon la revendication 1, dans lequel l'enzyme de type cellulasique provient de l'espèce Trichoderma.

3. Procédé selon la revendication 2, dans lequel l'enzyme de type cellulasique a une activité carboxyméthyle-cellulosique d'au moins 3 600 U/ml ou plus, lequel enzyme est obtenu à partir de Trichoderma Longibrachiatum.

4. Préparation selon la revendication 1, dans laquelle l'enzyme de type cellulasique est ajouté à la pâte en une quantité de 0,01-0.2% du poids de la pâte.

5. Procédé selon la revendication 1, dans lequel la charge inorganique est une au moins qui sera choisie dans un groupe consistant en talc, carbonate de calcium et rutile.

6. Procédé selon la revendication 1, dans lequel la charge inorganique est présente dans une quantité de 30-40% de poids.
